# EUROPEAN PATENT SPECIFICATION

(11) **EP 3 425 036 B1**
(45) Date of publication and mention of the grant of the patent: **05.01.2022**
(21) Application number: 18173618.2
(22) Date of filing: 22.05.2018
(51) Int. Cl.: A01N 25/28, A23L 27/00, A61K 9/50, B01J 13/14, B01J 13/20, C11D 3/37, C11D 17/00, C11D 3/50, C12N 9/10, D06M 13/00, D06M 23/12

(54) **BRANCHED POLYETHYLENEIMINE MICROCAPSULES**
VERZWEIGTE POLYETHYLENIMINMIKROKAPSELN
MICROCAPSULES DE POLYÉTHYLÈNEIMINE RAMIFIÉES

(30) Priority: 30.05.2017 US 201715607922
(43) Date of publication of application: 09.01.2019
(73) Proprietor: International Flavors & Fragrances Inc., New York, NY 10019 (US)
(72) Inventor: POPPLEWELL, Lewis Michael, Morganville, NJ New Jersey 07751 (US); LEI, Yabin, Holmdel, NJ New Jersey 07733 (US); BRAHMS, John, Morris Plains, NJ New Jersey 07950 (US); XU, Li, Edison, NJ New Jersey 08817 (US); FEI, Xiang, Edison, NJ New Jersey 08817 (US)
(74) Representative: Barker Brettell LLP

(56) References cited:
- WO-A1-2016/054351
- WO-A1-2018/006089
- US-A1- 2016 158 121
- Anonymous: "branched PEI", SIGMA-ALDRICH - Materials Science , 2016, XP002786294, Retrieved from the Internet: URL:https://www.sigmaaldrich.com/materials -science/material-science-products.html?Ta blePage=112434557 [retrieved on 2018-11-06]

## Description

### BACKGROUND

Nano- or micro-encapsulation is used in a variety of different applications where there is a need to deliver, apply, or release a fragrance or other active material at all stages of use in a time-delayed or controlled manner.

Aminoplast and polyurea microcapsules have been developed to provide good performance in laundry applications. See US 6,261,483, US 2014/0287008, and WO 2015/023961. Preparation of an aminoplast microcapsule almost always involves use of formaldehyde. A polyurea microcapsule is typically prepared from a polyisocyanate. Unreacted formaldehyde and polyisocyanate inevitably remain in the final product. Due to regulatory and/or environmental concerns, it is desirable to reduce or avoid the use of both formaldehyde and the polyisocyanate.

In an effort to eliminate the use of formaldehyde, US 8,835,002 and US 2014/0322283 describe a microcapsule essentially free of formaldehyde prepared from melamine. However, its performance or stability is problematic in certain applications

Microcapsules obtained by coacervation of branched polyethyleneimine (BPEI) with anionic polymer are described in US2016/158121 and WO2016/054351.

There is a need to develop a microcapsule with high performance and stability for delivering and releasing a fragrance in a controlled manner.

### SUMMARY

This invention is based on the discovery that branched polyethyleneimine ("BPEI") microcapsules show high performance and great stability.

Accordingly, one aspect of this invention relates to a microcapsule comprising an oil core and a microcapsule wall encapsulating the oil core, wherein the oil core contains an active material, the microcapsule wall is formed of an encapsulating polymer that is a reaction product of (i) a branched polyethyleneimine, a mixture of the branched polyethyleneimine and a polyfunctional amine, or a mixture of the branched polyethyleneimine and a polyfunctional alcohol, and (ii) a carbonyl crosslinker, or a mixture of a carbonyl crosslinker and a polyisocyanate, the carbonyl crosslinker has a first functional group and a second functional group, the first functional group is a carbonyl electrophilic group reactive towards the branched polyethyleneimine, the polyfunctional amine, or the polyfunctional alcohol, the second functional group is an electrophilic group reactive towards the branched polyethyleneimine, the polyfunctional amine, or the polyfunctional alcohol, the branched polyethyleneimine has a molecular weight of 750 to 50,000 Daltons, the carbonyl crosslinker has a molecular weight of 50 to 2,500 Daltons, the molar ratio between the branched polyethyleneimine and the carbonyl crosslinker is 1 : 20 to 20 : 1, the first functional group is formyl, an acyl halide group, a carboxylic anhydride group, and the second functional group is formyl, keto, carboxyl, a carboxylate ester group, an acyl halide group, an amide group, a carboxylic anhydride group, an alkyl halide group, an epoxide group, an aziridine group, an oxetane group, an azetidine group, a sulfonyl halide group, a chlorophosphate group, an isocyanate group, an α,β-unsaturated carbonyl group, an α,β-unsaturated nitrile group, or an α,β-unsaturated methanesulfonyl group.

The active material may be a fragrance, pro-fragrance, flavor, vitamin or derivative thereof, malodor counteractive agent, anti-inflammatory agent, fungicide, anesthetic, analgesic, antimicrobial active, anti-viral agent, anti-infectious agent, anti-acne agent, skin lightening agent, insect repellant, emollient, skin moisturizing agent, wrinkle control agent, UV protection agent, fabric softener active, hard surface cleaning active, skin or hair conditioning agent, insect repellant, animal repellent, vermin repellent, flame retardant, antistatic agent, nanometer to micron size inorganic solid, polymeric or elastomeric particle, and combinations thereof.

In some embodiments, the molar ratio between the BPEI and the carbonyl crosslinker is 1 : 10 to 1 : 1).

In some embodiments, the carbonyl crosslinker has a molecular weight of 55 to 2,000 Daltons.

In some embodiments, the carbonyl crosslinker has at least two formyl groups. Examples include dialdehydes such as glyoxal, 1,3-propane dialdehyde, 1,4-butane dialdehyde, 1,5-pentane dialdehyde, and 1,6-hexane dialdehyde, starch aldehyde, and combinations thereof.

In one embodiment, the microcapsule wall is formed of an encapsulating polymer that is the reaction product of (i) the branched polyethyleneimine and (ii) the carbonyl crosslinker.

In another embodiment, the microcapsule wall is formed of an encapsulating polymer that is the reaction product of (i) the branched polyethyleneimine and (ii) the mixture of the carbonyl crosslinker and the polyisocyanate.

In any of the microcapsules described above, a deposition aid can also be included. Nonlimiting examples of the deposition aid are polyquaternium-4, polyquaternium-5, polyquaternium-6, polyquaternium-7, polyquaternium-10, polyquaternium-16, polyquaternium-22, polyquaternium-24, polyquaternium-28, polyquaternium-39, polyquaternium-44, polyquaternium-46, polyquaternium-47, polyquaternium-53, polyquaternium-55, polyquaternium-67, polyquaternium-68, polyquaternium-69, polyquaternium-73, polyquaternium-74, polyquaternium-77, polyquaternium-78, polyquaternium-79, polyquaternium-80, polyquaternium-81, polyquaternium-82, polyquaternium-86, polyquaternium-88, polyquaternium-101, polyvinylamine, polyethyleneimine, polyvinylamine and vinylformamide copolymer, and any combinations thereof.

Another aspect of this invention relates to a method of preparing a microcapsule as defined in claim 1, the method comprising: (a) providing an oil-in-water emulsion containing (i) a branched polyethyleneimine, a mixture of the branched polyethyleneimine and a polyfunctional amine, or a mixture of the branched polyethyleneimine and a polyfunctional alcohol, (ii) a carbonyl crosslinker, or a mixture of the carbonyl crosslinker and a polyisocyanate, (iii) an oil phase having an active material, and (iv) an aqueous phase having a microcapsule formation aid and water, (b) causing the formation of a microcapsule precursor having an oil core that contains the active material and a microcapsule wall that is formed of an encapsulating polymer, and (c) curing the microcapsule precursor to obtain a microcapsule slurry containing the microcapsule, wherein the carbonyl crosslinker contains a first functional group and a second functional group, the first functional group is a carbonyl electrophilic group, the second functional group is an electrophilic group, the branched polyethyleneimine having a molecular weight of 750 to 25,000, the carbonyl crosslinker has a molecular weight of 50 to 2,500, the molar ratio between the polyfunctional amine and the carbonyl crosslinker is 1 : 20 to 20 : 1, the first functional group is formyl, an acyl halide group, a carboxylic anhydride group, the second functional group is formyl, keto, carboxyl, a carboxylate ester group, an acyl halide group, an amide group, a carboxylic anhydride group, an alkyl halide group, an epoxide group, an aziridine group, an oxetane group, an azetidine group, a sulfonyl halide group, a chlorophosphate group, an isocyanate group, an α,β-unsaturated carbonyl group, an α,β-unsaturated nitrile group, or an α,β-unsaturated methanesulfonyl group, the microcapsule formation aid is polyvinyl pyrrolidone, polyvinyl alcohol, poly(styrene sulfonate), carboxymethyl cellulose, sodium salt of naphthalene sulfonate condensate, co-polymer of ethylene and maleic anhydride, an alginate, hyaluronic acid, poly(acrylic acid), carboxymethylecellulose, copolymers of acrylic acid and acrylamide, copolymer of acrylamide and acrylamidopropyltrimonium chloride, terpolymers of (acrylic acid, acrylamide, and acrylamidopropyltrimonium chloride), partially or completely hydrolyzed polyvinyl acetate polymers, or a combination thereof, and the microcapsule precursor is cured at a temperature of 20 to 250 °C.

In one embodiment, the microcapsule precursor is cured at a temperature of 35 to 145 °C.

In one embodiment, the method further includes either or any combination of the following step: (i) adding a deposition aid to the microcapsule slurry, (ii) washing the microcapsule slurry with water, (iii) spray drying the microcapsule slurry to obtain the microcapsule in a powder form.

Still yet within the scope of this invention are consumer products containing any of the microcapsules described above. The consumer products include hair care products such as shampoos and hair conditioners, personal care products for example bar soaps, fabric care products include detergents, fabric conditioners, fabric refresher and the like, and home care products.

All parts, percentages and proportions refer to herein and in the claims are by weight unless otherwise indicated.

The terms "capsule" and "microcapsule" herein are used interchangeably.

The terms "polyfunctional isocyanate," "multifunctional isocyanate," and "polyisocyanate" all refer to a compound having two or more isocyanate (-NCO) groups.

The terms "polyfunctional amine," "multifunctional amine," and "polyamine" refers to a compound containing two or more primary or secondary amine groups. These terms also refers to a compound containing one or more primary/secondary amine groups and one or more hydroxyl groups (-OH).

The terms "polyfunctional alcohol," "multifunctional alcohol," "poly alcohol," and "polyol" refer to a compound having two or more hydroxyl groups.

The term "carbonyl electrophile" refers to a chemical moiety having a carbonyl group that is attracted to an electron rich center and is capable of receiving a pair of electrons to make a new covalent bond.

The term "carbonyl" refers to -C(O)-. Examples include formyl, keto, carboxyl, carboxylate ester, acyl halide, amide, and carboxylic anhydride.

The term "formyl" refers to -C(O)H connecting to an aliphatic, heteroaliphatic, aryl, or heteroaryl group.

The term "keto" refers to -C(O)- connecting to an aliphatic, heteroaliphatic, aryl, or heteroaryl group.

The term "carboxyl" refers to -C(O)OH or -C(O)O⁻ connecting to an aliphatic, heteroaliphatic, aryl, or heteroaryl group.

The term "carboxylate ester" refers to -C(O)OR^{a} connecting to an aliphatic, heteroaliphatic, aryl, or heteroaryl group, in which R^{a} is an aliphatic, heteroaliphatic, aryl, or heteroaryl group.

The term "acyl halide" refers to -C(O)X connecting to an aliphatic, heteroaliphatic, aryl, or heteroaryl group, in which X is F, Cl, Br, or I, preferably Cl or Br.

The term "amide" refers to -C(O)NR^{b}R^{c} connecting to an aliphatic, heteroaliphatic, aryl, or heteroaryl group, in which each of R^{b} and R^{c}, independently, is an aliphatic, heteroaliphatic, aryl, or heteroaryl group.

The term "carboxylic anhydride" refers to -C(O)OC(O)- connecting to two aliphatic, heteroaliphatic, aryl, or heteroaryl groups.

The term "alkyl halide" refers to -R^{d}X, in which R^{d} is alkyl and X is F, Cl, Br, or I (preferably Cl or Br).

The term "epoxide" refers to a three-membered ring formed of two carbon atoms and an oxygen atom.

The term "aziridine" refers to a three-membered ring formed of two carbon atoms and a nitrogen atom.

The term "oxetane" refers to a four-membered ring formed of three carbon atoms and an oxygen atom.

The term "azetidine" refers to a four-membered ring formed of three carbon atoms and a nitrogen atom.

The term "sulfonyl halide" refers to -SO₂X connecting to an aliphatic, heteroaliphatic, aryl, or heteroaryl group, in which X is F, Cl, Br, or I (preferably Cl or Br).

The term "chlorophosphate" refers to -OP(O)(Cl)O- connecting to two aliphatic, heteroaliphatic, aryl, or heteroaryl groups.

The term "isocyanate" refers to -NCO connecting to an aliphatic, heteroaliphatic, aryl, or heteroaryl group.

The term "α,β-unsaturated carbonyl" refers to -CR^{e}=CR^{f}-C(O)-, in which CR^{e} connects to an aliphatic, heteroaliphatic, aryl, or heteroaryl group; C(O)- connects to H, aliphatic, heteroaliphatic, aryl, heteroaryl, alkoxy, or amino; and each of R^{e} and R^{f}, independently, is aliphatic, heteroaliphatic, aryl, or heteroaryl.

The term "α,β-unsaturated nitrile" refers to -CR^{g}=CR^{h}-CN, in which CR^{g} connects to an aliphatic, heteroaliphatic, aryl, or heteroaryl group; and each of R^{g} and R^{h}, independently, is aliphatic, heteroaliphatic, aryl, or heteroaryl.

The term "α,β-unsaturated methanesulfonyl" refers to -CRⁱ=CR^{j}-SO₂CH₃ connecting to an aliphatic, heteroaliphatic, aryl, or heteroaryl group.

The term "aliphatic" herein refers to a saturated or unsaturated, linear or branched, acyclic, cyclic, or polycyclic hydrocarbon moiety. Examples include, but are not limited to, alkyl, alkylene, alkenyl, alkenylene, alkynyl, alkynylene, cycloalkyl, cycloalkylene, cycloalkenyl, cycloalkenylene, cycloalkynyl, and cycloalkynylene moieties. The term "alkyl" or "alkylene" refers to a saturated, linear or branched hydrocarbon moiety, such as methyl, methylene, ethyl, ethylene, propyl, propylene, butyl, butylenes, pentyl, pentylene, hexyl, hexylene, heptyl, heptylene, octyl, octylene, nonyl, nonylene, decyl, decylene, undecyl, undecylene, dodecyl, dodecylene, tridecyl, tridecylene, tetradecyl, tetradecylene, pentadecyl, pentadecylene, hexadecyl, hexadecylene, heptadecyl, heptadecylene, octadecyl, octadecylene, nonadecyl, nonadecylene, icosyl, icosylene, triacontyl, and triacotylene. The term "alkenyl" or "alkenylene" refers to a linear or branched hydrocarbon moiety that contains at least one double bond, such as -CH=CH-CH₃ and -CH=CH-CH₂-. The term "alkynyl" or "alkynylene" refers to a linear or branched hydrocarbon moiety that contains at least one triple bond, such as -C≡C-CH₃ and -C≡C-CH₂-. The term "cycloalkyl" or "cycloalkylene" refers to a saturated, cyclic hydrocarbon moiety, such as cyclohexyl and cyclohexylene. The term "cycloalkenyl" or "cycloalkenylene" refers to a non-aromatic, cyclic hydrocarbon moiety that contains at least one double bond, such as cyclohexenyl cyclohexenylene. The term "cycloalkynyl" or "cycloalkynylene" refers to a non-aromatic, cyclic hydrocarbon moiety that contains at least one triple bond, cyclooctynyl and cyclooctynylene.

The term "heteroaliphatic" herein refers to an aliphatic moiety containing at least one heteroatom selected from N, O, P, B, S, Si, Sb, Al, Sn, As, Se, and Ge.

The term "aryl" herein refers to a C₆ monocyclic, C₁₀ bicyclic, C₁₄ tricyclic, C₂₀ tetracyclic, or C₂₄ pentacyclic aromatic ring system. Examples of aryl groups include, but are not limited to, phenyl, phenylene, naphthyl, naphthylene, anthracenyl, anthracenylene, pyrenyl, and pyrenylene. The term "heteroaryl" herein refers to an aromatic 5-8 membered monocyclic, 8-12 membered bicyclic, 11-14 membered tricyclic, and 15-20 membered tetracyclic ring system having one or more heteroatoms (such as O, N, S, or Se). Examples of heteroaryl groups include, but are not limited to, furyl, furylene, fluorenyl, fluorenylene, pyrrolyl, pyrrolylene, thienyl, thienylene, oxazolyl, oxazolylene, imidazolyl, imidazolylene, benzimidazolyl, benzimidazolylene, thiazolyl, thiazolylene, pyridyl, pyridylene, pyrimidinyl, pyrimidinylene, quinazolinyl, quinazolinylene, quinolinyl, quinolinylene, isoquinolyl, isoquinolylene, indolyl, and indolylene.

Unless specified otherwise, aliphatic, heteroaliphatic, oxyaliphatic, alkyl, alkylene, alkenyl, alkenylene, alkynyl, alkynylene, cycloalkyl, cycloalkylene, cycloalkenyl, cycloalkenylene, cycloalkynyl, cycloalkynylene, heterocycloalkyl, heterocycloalkylene, heterocycloalkenyl, heterocycloalkenylene, aryl, and heteroaryl mentioned herein include both substituted and unsubstituted moieties. Possible substituents on cycloalkyl, cycloalkylene, cycloalkenyl, cycloalkenylene, cycloalkynyl, cycloalkynylene, heterocycloalkyl, heterocycloalkylene, heterocycloalkenyl, heterocycloalkenylene, aryl, and heteroaryl include, but are not limited to, C₁-C₁₀ alkyl, C₂-C₁₀ alkenyl, C₂-C₁₀ alkynyl, C₃-C₂₀ cycloalkyl, C₃-C₂₀ cycloalkenyl, C₃-C₂₀ heterocycloalkyl, C₃-C₂₀ heterocycloalkenyl, C₁-C₁₀ alkoxy, aryl, aryloxy, heteroaryl, heteroaryloxy, amino, C₁-C₁₀ alkylamino, C₂-C₂₀ dialkylamino, arylamino, diarylamino, C₁-C₁₀ alkylsulfonamino, arylsulfonamino, C₁-C₁₀ alkylimino, arylimino, C₁-C₁₀ alkylsulfonimino, arylsulfonimino, hydroxyl, halo, thio, C₁-C₁₀ alkylthio, arylthio, C₁-C₁₀ alkylsulfonyl, arylsulfonyl, acylamino, aminoacyl, aminothioacyl, amido, amidino, guanidine, ureido, thioureido, cyano, nitro, nitroso, azido, acyl, thioacyl, acyloxy, carboxyl, and carboxylic ester. On the other hand, possible substituents on aliphatic, heteroaliphatic, oxyaliphatic, alkyl, alkylene, alkenyl, alkenylene, alkynyl, and alkynylene include all of the above-recited substituents except C₁-C₁₀ alkyl. Cycloalkyl, cycloalkylene, cycloalkenyl, cycloalkenylene, heterocycloalkyl, heterocycloalkylene, heterocycloalkenyl, heterocycloalkenylene, aryl, and heteroaryl can also be fused with each other.

The details of one or more embodiments of the invention are set forth in the description below. Other features, objects, and advantages of the invention will be apparent from the description and the claims.

### DETAILED DESCRIPTION

It has been found that BPEI microcapsules are suitable for delivering active materials such as fragrances in consumer products.

These BPEI microcapsules find their utility in a wide range of consumer applications, e.g., personal care products including shampoos, hair conditioners, hair rinses, hair refreshers; personal wash such as bar soaps, body wash, personal cleaners and sanitizers, hydro-alcoholic formulations; fabric care such as fabric refreshers, softeners and dryer sheets, ironing water, industrial cleaners, liquid and powder detergent including unit dose capsules, rinse conditioners, and scent booster products; fine fragrances; an Eau De Toilette products; deodorants; roll-on products, and aerosol products.

The BPEI microcapsules preferably have a size in the range of from 0.01 to 1000 microns in diameter (e.g., 0.5 to 1000 microns, 1 to 200 microns, 0.5 to 150 microns, 0.1 to 100 microns, 2 to 50 microns, 5 to 25 microns, 2 to 15 microns, and 1 to 10 microns). The capsule distribution can be narrow, broad, or multi-modal.

The BPEI microcapsules of this invention each include an oil core and a capsule wall encapsulating the oil core.

The oil core contains an active material, which is preferably a fragrance, flavor, malodor counteractive agent, a cosmetic active, or a combination thereof. The active material can be present at a level of 5 to 95% (preferably 20 to 90% and more preferably 40 to 85%) by weight of the microcapsule.

In one aspect, the microcapsule wall is formed of an encapsulating polymer that is a reaction product of a BPEI and a carbonyl crosslinker.

The BPEI can be used free of any other polyamine or polyalcohol. It can also be used together with one or more polyamines and/or polyalcohols.

The carbonyl crosslinker can be used by itself. It can also be used together with one or more polyisocyanates and/or one or more other carbonyl crosslinker.

In another aspect, the microcapsule wall is formed of an encapsulating polymer that is an aggregate of a BPEI. In some embodiments, the aggregation consists essential of BPEI, e.g., by adjusting the pH of the BPEI solution so that it assembles into an aggregate to encapsulate the oil core.

In other embodiments, the aggregate is formed when an aggregate formation aid is added to BPEI to form the aggregate. In these embodiments, the aggregate contains BPEI and an aggregate formation aid. Suitable aggregate formation aids include water-soluble cations, water-soluble anions, and a transglutaminase. Preferred aggregate formation aids are the transglutaminase, multivalent water-soluble anions (e.g., sulfate, carbonate, and phosphate) and anionic polymers (such as an alginate, poly(styrene sulfonate), hyaluronic acid, poly(acrylic acid), carboxymethylecellulose, gelatin, and combinations thereof).

Some of the above components are described in detail below.

### BPEI

Representative BPEI structure is shown below: 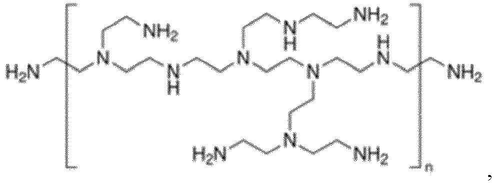 in which n is an integer from 1 to 20,000 *(e.g.,* 1 to 10,000, 2 to 5,000, and 2 to 1,000). BPEI for use in this disclosure preferably has a molecular weight of 500 to 5,000,000 Daltons *(e.g.,* 500 to 1,000,000 Daltons, 750 to 500,000 Daltons, 750 to 100,000 Daltons, and 750 to 50,000 Daltons).

BPEI are commercially available from Sigma-Aldrich (St. Louis, MO; average molecular weight 25,000 Daltons) and Polysciences Inc. (Warrington, PA; various products having molecular weight from 600, 1200, 1800, 10,000, 70,000, 750,000, 250,000, and 2,000,000 Daltons).

### Polyfunctional amines

Suitable polyfunctional amines include those described in WO 2015/023961. Examples are hexamethylenediamine, hexaethylenediamine, ethylenediamine, 1,3-diaminopropane, 1,4-diaminobutane, diethylenetriamine, pentaethylenehexamine, bis(3-aminopropyl)amine, bis(hexanethylene)triamine, tris(2-aminoethyl)amine, triethylene-tetramine, N,N'-bis(3-aminopropyl)-1,3-propanediamine, tetraethylenepentamine, amino-2-methyl-1-propanol, chitosan, 1,3-diamino-guanidine, 1,1-dimethylbiguanide, guanidine, arginine, lysine, histidine, ornithine, nisin, gelatin, and combinations thereof.

### Polyfunctional alcohols

Suitable polyfunctional alcohols are also described in WO 2015/023961. Examples include pentaerythritol, dipentaerythritol, glycerol, polyglycerol, ethylene glycol, polyethylene glycol, trimethylolpropane, neopentyl glycol, sorbitol, erythritol, threitol, arabitol, xylitol, ribitol, mannitol, galactitol, fucitol, iditol, inositol, volemitol, isomalt, maltitol, lactitol, maltotriitol, maltotetraitol, polyglycitol, and combinations thereof.

### Carbonyl crosslinkers

The carbonyl crosslinkers each have at least two functional groups, *e.g.,* a first functional group and a second functional group.

The first functional group is an electrophilic group reactive towards the branched polyethyleneimine, the polyfunctional amine, or the polyfunctional alcohol to form a network of the encapsulating polymer. Examples include formyl, keto, carboxyl, a carboxylate ester group, an acyl halide group, an amide group, a carboxylic anhydride group, an alkyl halide group, an epoxide group, an aziridine group, an oxetane group, an azetidine group, a sulfonyl halide group, a chlorophosphate group, an isocyanate group, an α,β-unsaturated carbonyl group, an α,β-unsaturated nitrile group, or an α,β-unsaturated methanesulfonyl group. Preferably, the first function group is a carbonyl electrophilic group containing a carbonyl group such as formyl, keto, carboxyl, a carboxylate ester group, an acyl halide group, an amide group, a carboxylic anhydride group, an α,β-unsaturated carbonyl group, a trifluoromethanesulfonate group, and a *p*-toluenesulfonate group.

The second functional group is an electrophilic group reactive towards the branched polyethyleneimine, the polyfunctional amine, or the polyfunctional alcohol. It can be selected from the groups listed immediately above.

Examples of a carbonyl crosslinker include glutaric dialdehyde, succinic dialdehyde, and glyoxal; as well as compounds such as glyoxyl trimer and paraformaldehyde, bis(dimethyl) acetal, bis(diethyl) acetal, polymeric dialdehydes, such as oxidized starch. Preferably the cross-linking agent is a low molecular weight, difunctional aldehyde, such as glyoxal, 1,3-propane dialdehyde, 1,4-butane dialdehyde, 1,5-pentane dialdehyde, or 1,6-hexane.

Some crosslinking reactions between the BPEI and the carbonyl crosslinker are shown below:

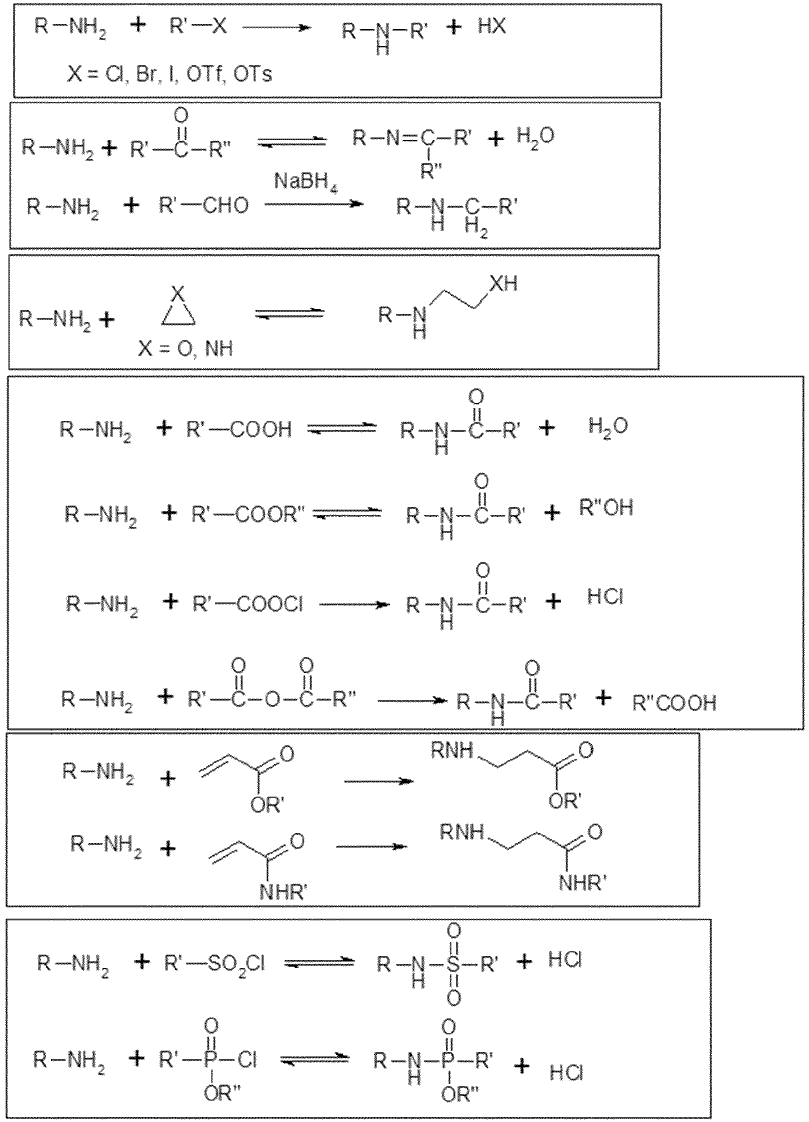

In the scheme above, R-NH₂ is BPEI, and the other reactant is the carbonyl crosslinker. In some of the above reactions, a C=N double bond is formed, which can be reduced to a more stable C-N single bond by a reducing agent, e.g., sodium borohydride.

More examples of the crosslinking reactions are shown below: 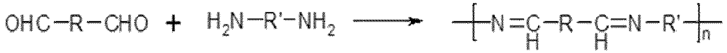 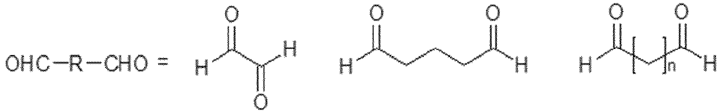 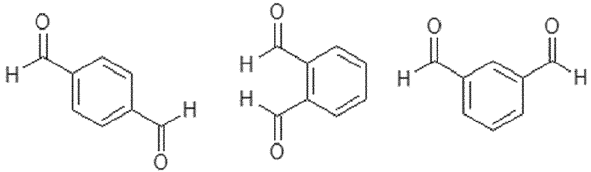 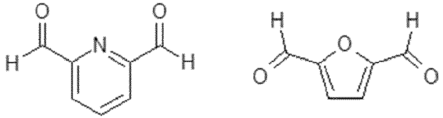 etc

H₂N-R'-NH₂ = BPEI

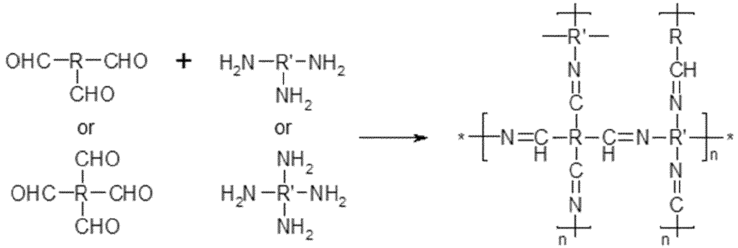

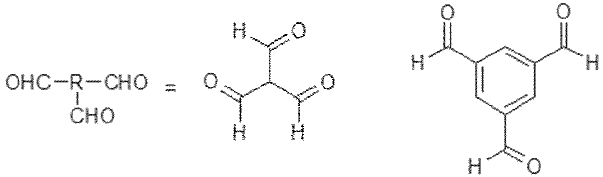

etc

### Polyisocyanates

The encapsulating polymer can be a reaction product between BPEI (or its mixture) and a mixture of a carbonyl crosslinker and a polyisocyanate.

These polyisocyanates each contain two or more isocyanate (-NCO) groups. Suitable polyisocyanates include, for example, 1,5-naphthylene diisocyanate, 4,4'-diphenylmethane diisocyanate (MDI), hydrogenated MDI (H12MDI), xylylene diisocyanate (XDI), tetramethylxylol diisocyanate (TMXDI), 4,4'-diphenyldimethylmethane diisocyanate, di- and tetraalkyldiphenylmethane diisocyanate, 4,4'-dibenzyl diisocyanate, 1,3-phenylene diisocyanate, 1,4-phenylene diisocyanate, the isomers of tolylene diisocyanate (TDI), optionally in a mixture, 1-methyl-2,4-diisocyanatocyclohexane, 1,6-diisocyanato-2,2,4-trimethylhexane, 1,6-diisocyanato-2,4,4-trimethylhexane, 1-isocyanatomethyl-3-isocyanato-1,5,5-trimethylcyclohexane, chlorinated and brominated diisocyanates, phosphorus-containing diisocyanates, 4,4'-diisocyanatophenylperfluoroethane, tetramethoxybutane 1,4-diisocyanate, butane 1,4-diisocyanate, hexane 1,6-diisocyanate (HDI), dicyclohexylmethane diisocyanate, cyclohexane 1,4-diisocyanate, ethylene diisocyanate, phthalic acid bisisocyanatoethyl ester, also polyisocyanates with reactive halogen atoms, such as 1-chloromethylphenyl 2,4-diisocyanate, 1-bromomethylphenyl 2,6-diisocyanate, and 3,3-bischloromethyl ether 4,4'-diphenyldiisocyanate. Sulfur-containing polyisocyanates are obtained, for example, by reacting hexamethylene diisocyanate with thiodiglycol or dihydroxydihexyl sulfide. Further suitable diisocyanates are trimethylhexamethylene diisocyanate, 1,4-diisocyanatobutane, 1,2-diisocyanatododecane, dimer fatty acid diisocyanate, and combinations thereof.

Other suitable commercially-available polyisocyanates include LUPRANATE M20 (PMDI, commercially available from BASF containing isocyanate group "NCO" 31.5 wt%), where the average n is 0.7; PAPI 27 (PMDI commercially available from Dow Chemical having an average molecular weight of 340 and containing NCO 31.4 wt%) where the average n is 0.7; MONDUR MR (PMDI containing NCO at 31 wt% or greater, commercially available from Bayer) where the average n is 0.8; MONDUR MR Light (PMDI containing NCO 31.8 wt%, commercially available from Bayer) where the average n is 0.8; MONDUR 489 (PMDI commercially available from Bayer containing NCO 30-31.4 wt%) where the average n is 1.0; poly[(phenylisocyanate)-co-formaldehyde] (Aldrich Chemical, Milwaukee, WI), other isocyanate monomers such as DESMODUR N3200 (poly(hexamethylene diisocyanate) commercially available from Bayer), and TAKENATE D110-N (xylene diisocyanate adduct polymer commercially available from Mitsui Chemicals corporation, Rye Brook, NY, containing NCO 11.5 wt%), DESMODUR L75 (a polyisocyanate base on toluene diisocyanate commercially available from Bayer), and DESMODUR IL (another polyisocyanate based on toluene diisocyanate commercially available from Bayer).

In some embodiments, the polyisocyanate used in the preparation of the capsules of this disclosure is a single polyisocyanate. In other embodiments the polyisocyanate is a mixture of polyisocyanates. In some embodiments, the mixture of polyisocyanates includes an aliphatic polyisocyanate and an aromatic polyisocyanate. In particular embodiments, the mixture of polyisocyanates is a biuret of hexamethylene diisocyanate and a trimethylol propane-adduct of xylylene diisocyanate. In certain embodiments, the polyisocyanate is an aliphatic isocyanate or a mixture of aliphatic isocyanate, free of any aromatic isocyanate. In other words, in these embodiments, no aromatic isocyanate is used to prepare the polyurea/polyureathane polymers as capsule wall materials.

The average molecular weight of certain suitable polyisocyanates varies from 250 to 1000 Da and preferable from 275 to 500 Da. In general, the range of the polyisocyanate concentration varies from 0.1% to 10%, preferably from 0.1% to 8%, more preferably from 0.2 to 5%, and even more preferably from 1.5% to 3.5%, all based on the weight of the capsule delivery system.

More examples of suitable polyisocyanates can be found in WO 2004/054362; WO 2015/023961; EP 0 148149; EP 0 017 409 B1; U.S. Pat. No. 4,417,916, U.S. Pat. No. 4,124,526, U.S. Pat. No. 5,583,090, U.S. Pat. No. 6,566,306, U.S. Pat. No. 6,730,635, PCT 90/08468, PCT WO 92/13450, U.S. Pat. No. 4,681,806, U.S. Pat. No. 4,285,720 and U.S. Pat. No. 6,340,653.

### Additional wall polymer

The Encapsulating polymer can also include one or more additional wall polymers, e.g., a second, third, fourth, fifth, or sixth polymer. These additional polymers can be selected from the group consisting of silica, polyacrylate, polyacrylamide, poly(acrylate-co-acrylamide), polyurea, polyurethane, starch, gelatin and gum Arabic, poly(melamine-formaldehyde), poly(urea-formaldehyde), and combinations thereof.

### Encapsulation methods

Conventional encapsulation methods can be used to prepare the BPEI microcapsules. See WO 2015/023961. In some embodiments, capsule formation aids, *e.g.,* a surfactant or dispersant, are used.

By way of illustration, to prepare a BPEI microcapsule having a crosslinked encapsulating polymer, an oil-in-water emulsion is first prepared containing (i) BPEI or its mixture, (ii) a carbonyl crosslinker or its mixture, (iii) an oil phase having an active material, and (iv) an aqueous phase having a microcapsule formation aid and water. The reaction between BPEI (or its mixture) and the carbonyl crosslinker (or its mixture) occurs when the temperature of the reaction mixture is raised or a catalyst (such as a transglutaminase for catalyzing amide formation) is added to the mixture.

Catalysts suitable for use in the present disclosure are transglutaminases, metal carbonates, metal hydroxide, amino or organometallic compounds and include, for example, sodium carbonate, cesium carbonate, potassium carbonate, lithium hydroxide, 1,4-diazabicyclo[2.2.2]octane *(i.e.,* DABCO), N,N-dimethylaminoethanol, N,N-dimethylcyclohexylamine, bis-(2-dimethylamino-ethyl) ether, N,N dimethylacetylamine, stannous octoate and dibutyltin dilaurate.

The resultant microcapsule slurry is then cured at a predetermined temperature for a predetermined period of time.

To prepare a BPEI microcapsule having an aggregate of BPEI, an oil-in-water emulsion is first prepared by emulsifying (i) a branched polyethyleneimine, (ii) an oil phase having an active material, and (iii) an aqueous phase having a microcapsule formation aid and water. The aggregate is then formed by adjusting the pH of the emulsion, raising the temperature of the emulsion, or adding an aggregate formation aid to the emulsion. The resultant microcapsule slurry is then cured to make the BPEI microcapsule having the BPEI aggregate.

In accordance with some embodiments of this disclosure, the microcapsules prepared according to the methods above are cured at a temperature in the range of, *e.g.,* 15°C to 230°C (e.g., 55°C to 90°C, 55°C to 75°C, and 90°C to 130°C) for 1 minute to 10 hours (e.g., 0.1 hours to 5 hours, 0.2 hours to 4 hours and 0.5 hours to 3 hours). A skilled person in the art can determine, without undue experiments, the curing temperature, duration, and the heating rate.

To obtain microcapsules with more leaching of the active material, certain embodiments of this disclosure provide for a cure at a low temperature, e.g., less than 100°C. In some embodiments, the cure temperature is at or less than 90°C. In other embodiments, the cure temperature is at or less than 80°C.

In one embodiment, the capsules are heated to a target cure temperature at a linear rate of 0.5 to 2 °C per minute (e.g., 1 to 5 °C per minute, 2 to 8 °C per minute, and 2 to 10°C per minute) over a period of 1 to 60 minutes (e.g., 1 to 30 minutes). The following heating methods may be used: conduction for example via oil, steam radiation via infrared, and microwave, convection via heated air, steam injection and other methods known by those skilled in the art. The target cure temperature used herein refers to the minimum temperature in degrees Celsius at which the capsules may be cured to retard leaching.

### Microcapsule formation aids

Most microcapsule formation aids are used as dispersants (namely, emulsifiers or surfactants). They facilitate the formation of stable emulsions containing nano- or micro-sized oil drops to be encapsulated. Further, microcapsule formation aids improve the performance of the microcapsule by stabilizing capsules and/or their deposition to the target areas or releasing to the environment. Performance is measured by the intensity of the fragrance release during the use experience, such as the pre-rub and post-rub phases in a laundry experience. The pre-rub phase is the phase when the microcapsules have been deposited on the cloth, e.g., after a fabric softener containing microcapsules has been used during the wash cycle. The post-rub phase is after the microcapsules have been deposited and the microcapsules are broken by friction or other similar mechanisms.

The amount of these microcapsule formation aids is anywhere from about 0.1 to about 40 percent by weight of the microcapsule, more preferably from 0.5 to about 10 percent, more preferably 0.5 to 5 percent by weigh.

Preferred microcapsule formation aids are polyvinyl pyrrolidone, polyvinyl alcohol, poly(styrene sulfonate), carboxymethyl cellulose, sodium salt of naphthalene sulfonate condensate, co-polymer of ethylene and maleic anhydride, an alginate, hyaluronic acid, poly(acrylic acid), carboxymethylcellulose, copolymers of acrylic acid and acrylamide, copolymer of acrylamide and acrylamidopropyltrimonium chloride, terpolymers of (acrylic acid, acrylamide, and acrylamidopropyltrimonium chloride), partially or completely hydrolyzed polyvinyl acetate polymers *(i.e.,* polyvinyl alcohol), and combinations thereof.

Other microcapsule formation aids include water-soluble salts of alkyl sulfates, alkyl ether sulfates, alkyl isothionates, alkyl carboxylates, alkyl sulfosuccinates, alkyl succinamates, alkyl sulfate salts such as sodium dodecyl sulfate, alkyl sarcosinates, alkyl derivatives of protein hydrolyzates, acyl aspartates, alkyl or alkyl ether or alkylaryl ether phosphate esters, sodium dodecyl sulphate, phospholipids or lecithin, or soaps, sodium, potassium or ammonium stearate, oleate or palmitate, alkylarylsulfonic acid salts such as sodium dodecylbenzenesulfonate, sodium dialkylsulfosuccinates, dioctyl sulfosuccinate, sodium dilaurylsulfosuccinate, poly(styrene sulfonate) sodium salt, isobutylene-maleic anhydride copolymer, gum arabic, sodium alginate, cellulose sulfate and pectin, isobutylene-maleic anhydride copolymer, gum arabic, carrageenan, sodium alginate, pectic acid, tragacanth gum, almond gum and agar; semi-synthetic polymers such as sulfated cellulose, sulfated methylcellulose, carboxymethyl starch, phosphated starch, lignin sulfonic acid; and synthetic polymers such as maleic anhydride copolymers (including hydrolyzates thereof), polyacrylic acid, polymethacrylic acid, acrylic acid butyl acrylate copolymer or crotonic acid homopolymers and copolymers, vinylbenzenesulfonic acid or 2-acrylamido-2-methylpropanesulfonic acid homopolymers and copolymers, and partial amide or partial ester of such polymers and copolymers, carboxymodified polyvinyl alcohol, sulfonic acid-modified polyvinyl alcohol and phosphoric acid-modified polyvinyl alcohol, phosphated or sulfated tristyrylphenol ethoxylates.

Commercially available surfactants include, but are not limited to, sulfonated naphthalene-formaldehyde condensates such as MORWET D425 (naphthalene sulfonate, Akzo Nobel, Fort Worth, TX); partially hydrolyzed polyvinyl alcohols such as MOWIOLs, e.g., MOWIOL 3-83 (Air Products); ethylene oxide-propylene oxide block copolymers or poloxamers such as PLURONIC, SYNPERONIC or PLURACARE materials (BASF); sulfonated polystyrenes such as FLEXAN II (Akzo Nobel); ethylene-maleic anhydride polymers such as ZEMAC (Vertellus Specialties Inc.); copolymer of acrylamide and acrylamidopropyltrimonium chloride such as Salcare SC 60 (BASF); and Polyquaternium series such as Polyquaternium 11 ("PQ11;" a copolymer of vinyl pyrrolidone and quaternized dimethylaminoethyl methacrylate; sold by BASF as LUVIQUAT PQ11 AT 1).

In other embodiments, the capsule formation aid is a processing aid such as hydrocolloids, which improve the colloidal stability of the slurry against coagulation, sedimentation and creaming. The term "hydrocolloid" refers to a broad class of water-soluble or water-dispersible polymers having anionic, cationic, zwitterionic or non-ionic character. Hydrocolloids useful in the present disclosure include, but are not limited to, polycarbohydrates, such as starch, modified starch, dextrin, maltodextrin, and cellulose derivatives, and their quaternized forms; natural gums such as alginate esters, carrageenan, xanthanes, agar-agar, pectines, pectic acid, and natural gums such as gum arabic, gum tragacanth and gum karaya, guar gums and quaternized guar gums; gelatine, protein hydrolysates and their quaternized forms; synthetic polymers and copolymers, such as poly(vinyl pyrrolidone-co-vinyl acetate), poly(vinyl alcohol-co-vinyl acetate), poly((met)acrylic acid), poly(maleic acid), poly(alkyl(meth)acrylate-co-(meth)acrylic acid), poly(acrylic acid-co-maleic acid)copolymer, poly(alkyleneoxide), poly(vinylmethylether), poly(vinylether-co-maleic anhydride), and the like, as well as poly-(ethyleneimine), poly((meth)acrylamide), poly(alkyleneoxide-co-dimethylsiloxane), poly(amino dimethylsiloxane), and the like, and their quaternized forms.

The capsule formation aid may also be used in combination with carboxymethyl cellulose ("CMC"), polyvinylpyrrolidone, polyvinyl alcohol, alkylnaphthalenesulfonate formaldehyde condensates, and/or a surfactant during processing to facilitate capsule formation. Examples of surfactants that can be used in combination with the capsule formation aid include, but are not limited to, cetyl trimethyl ammonium chloride (CTAC), poloxamers such as PLURONICS (e.g., PLURONIC F127), PLURAFAC (e.g., PLURAFAC F127), or MIRANET-N, saponins such as QNATURALE (National Starch Food Innovation); or a gum Arabic such as Seyal or Senegal. In certain embodiments, the CMC polymer has a molecular weight range between about 90,000 Daltons to 1,500,000 Daltons, preferably between about 250,000 Daltons to 750,000 Daltons and more preferably between 400,000 Daltons to 750,000 Daltons. The CMC polymer has a degree of substitution between about 0.1 to about 3, preferably between about 0.65 to about 1.4, and more preferably between about 0.8 to about 1.0. The CMC polymer is present in the capsule slurry at a level from about 0.1% to about 2% and preferably from about 0.3% to about 0.7%. in other embodiments, polyvinylpyrrolidone used in this disclosure is a water-soluble polymer and has a molecular weight of 1,000 to 10,000,000. Suitable polyvinylpyrrolidone are polyvinylpyrrolidone K12, K15, K17, K25, K30, K60, K90, or a mixture thereof. The amount of polyvinylpyrrolidone is 2-50%, 5-30%, or 10-25% by weight of the capsule delivery system. Commercially available alkylnaphthalenesulfonate formaldehyde condensates include MORWET D-425, which is a sodium salt of naphthalene sulfonate condensate by Akzo Nobel, Fort Worth, TX.

### Other delivery systems

The microcapsule compositions containing one of the BPEI microcapsules described above can also include one or more addition delivery systems.

In some embodiments, the microcapsule composition of this disclosure contains a BPEI microcapsule and one or more additional non-BPEI microcapsules. These microcapsules are free of BPEI. Wall forming materials include melamine formaldehyde, polyurethane, polysiloxanes, polyurea, polyamide, polyimide, polyvinyl alcohol, polyanhydride, polyolefin, polysulfone, polysaccharide, protein, polylactide (PLA), polyglycolide (PGA), polyorthoester, polyphosphazene, silicone, lipid, modified cellulose, gums, polystyrene, and polyesters or combinations of these materials. Other polymeric materials that are functional are ethylene maleic anhydride copolymer, styrene maleic anhydride copolymer, ethylene vinyl acetate copolymer, and lactide glycolide copolymer. Biopolymers that are derived from alginate, chitosan, collagen, dextran, gelatin, and starch can also be used as the encapsulating materials. Additionally, capsules can be made via the simple or complex coacervation of gelatin. Preferred encapsulating wall polymers include those formed from isocyanates, acrylates, acrylamide, acrylate-co-acrylamide, hydrogel monomers, sol-gel precursors, gelatin, melamine-formaldehyde or urea-formaldehyde condensates, as well as similar types of aminoplasts.

Preferred additional non-BPEI microcapsules are aminoplasts and gelatin capsules, urea-formaldehyde and melamine-formaldehyde capsules, and polyurea/polyurethane microcapsules. See US 2007/0078071, US 6,261,483, and US 8,299,011.

### Active Materials

The core of the capsules of the present disclosure can include one or more active materials including, but not limited to, flavors and/or fragrance ingredients such as fragrance oils. Nonlimiting examples include those described in WO 2016/049456. These active material include flavor or fragrance ingredients, taste masking agents, taste sensates, malodor counteracting agents, vitamins, antibacterials, sunscreen actives, antioxidants, anti-inflammatory agents, anesthetics, analgesics, antifungal agents, antibiotics, anti-viral agents, anti-parasitic agents, anti-infectious and anti-acne agents, dermatological active ingredients, enzymes and co-enzymes, skin whitening agents, anti-histamines, chemotherapeutic agents, and insect repellents. In addition to the active materials listed above, the products of this disclosure can also contain dyes, colorants or pigments, naturally obtained extracts (for example paprika extract and black carrot extract), and aluminum lakes.

In some embodiments, the amount of encapsulated active material is from 5 to 95% *(e.g.,* 20 to 90% and 40 to 85%) by weight of the capsule. The amount of the capsule wall is from 0.5% to 25% (e.g., 1.5 to 15% and 2.5 to 10%) also by weight of the capsule. In other embodiments, the amount of the encapsulated active material is from 15% to 99.5% (e.g., 50 to 98% and 30 to 95%) by weight of the capsule, and the amount of the capsule wall is from 0.5% to 85% (e.g., 2 to 50% and 5 to 70%) by weight of the capsule.

### Adjunct Materials

In addition to the active materials, the present disclosure also contemplates the incorporation of adjunct materials including solvent, emollients, and core modifier materials in the core encapsulated by the capsule wall. Other adjunct materials are nanoscale solid particulate materials, polymeric core modifiers, solubility modifiers, density modifiers, stabilizers, humectants, viscosity modifiers, pH modifiers, or any combination thereof. These modifiers can be present in the wall or core of the capsules, or outside the capsules in delivery system. Preferably, they are in the core as a core modifier.

The one or more adjunct material may be added in the amount of from 0.01% to 25% (e.g., from 0.5% to 10%) by weight of the capsule.

Suitable examples include those described in WO 2016/049456 and US 2016/0158121.

### Deposition Aid

A capsule deposition aid from 0.01 to 25%, more preferably from 5 to 20% can be included by weight of the capsule. The capsule deposition aid can be added during the preparation of the capsules or it can be added after the capsules have been made.

These deposition aids are used to aid in deposition of capsules to surfaces such as fabric, hair or skin. These include anionically, cationically, nonionically, or amphoteric water-soluble polymers. Suitable deposition aids include polyquaternium-4, polyquaternium-5, polyquaternium-6, polyquaternium-7, polyquaternium-10, polyquaternium-16, polyquaternium-22, polyquaternium-24, polyquaternium-28, polyquaternium-39, polyquaternium-44, polyquaternium-46, polyquaternium-47, polyquaternium-53, polyquaternium-55, polyquaternium-67, polyquaternium-68, polyquaternium-69, polyquaternium-73, polyquaternium-74, polyquaternium-77, polyquaternium-78, polyquaternium-79, polyquaternium-80, polyquaternium-81, polyquaternium-82, polyquaternium-86, polyquaternium-88, polyquaternium-101, polyvinylamine, polyethyleneimine, polyvinylamine and vinylformamide copolymer, an acrylamidopropyltrimonium chloride/acrylamide copolymer, a methacrylamidopropyltrimonium chloride/acrylamide copolymer, and combinations thereof. Other suitable deposition aids include those described in WO 2016049456, pages 13-27. Additional deposition aids are described in US 2013/0330292, US 2013/0337023, and US 2014/0017278.

### Capsule delivery system

The reloadable microcapsule can be formulated into a capsule delivery system (e.g., a microcapsule composition) for use in consumer products.

The capsule delivery system can be a slurry containing in an external hydrophilic solvent *(e.g.,* water, ethanol, and a combination thereof) the capsule at a level 0.1 to 80% *(e.g.,* 70-75%, 40-55%, 50-90%, 1 to 65%, and 5 to 45%) by weight of the capsule delivery system.

In some embodiments, the capsule and its slurry prepared in accordance with the present disclosure is subsequently purified. See US 2014/0017287. Purification can be achieved by washing the capsule slurry with water until a neutral pH is achieved.

The delivery system can also be spray dried to a solid form. In a spray drying process, a spray dry carrier is added to a capsule delivery system to assist the removal of water from the slurry. See WO 2016/144798.

The capsule delivery system can also be sprayed as a slurry onto a consumer product, *e.g.,* a fabric care product. By way of illustration, a liquid delivery system containing capsules is sprayed onto a detergent powder during blending to make granules. See US 2011/0190191. In order to increase fragrance load, water-absorbing material, such as zeolite, can be added to the delivery system.

Alternatively, granulates in a consumer product are prepared in a mechanical granulator in the presence of a granulation auxiliary such as non-acid water-soluble organic crystalline solids. See WO 2005/097962.
(iii) Additional components. The capsule delivery system can include one or more non-confined unencapsulated active materials from about 0.01% to about 50%, more preferably from about 5% to about 40%.

The capsule delivery system can also contain one or more other delivery system such as polymer-assisted delivery compositions (see US 8,187,580), fiber-assisted delivery compositions (US 2010/0305021), cyclodextrin host guest complexes (US 6,287,603 and US 2002/0019369), pro-fragrances (WO 2000/072816 and EP 0 922 084), and any combination thereof. The capsule delivery system can also contain one or more (e.g., two, three, four, five or six more) different capsules including different capsules of this disclosure and other capsules such as aminoplasts, hydrogel, sol-gel, coascervate capsules, polyurea/polyurethane capsules, and melamine formaldehyde capsules. More exemplary delivery systems that can be incorporated are coacervate capsules, cyclodextrin delivery systems, and spray dry encapsulation. See WO 2016/144798.

Any compound, polymer, or agent discussed above can be the compound, polymer, or agent itself as shown above, or its salt, precursor, hydrate, or solvate. A salt can be formed between an anion and a positively charged group on the compound, polymer, or agent. Suitable anions include chloride, bromide, iodide, sulfate, nitrate, phosphate, citrate, methanesulfonate, trifluoroacetate, acetate, malate, tosylate, tartrate, fumurate, glutamate, glucuronate, lactate, glutarate, and maleate. Likewise, a salt can also be formed between a cation and a negatively charged group on the compound, polymer, or agent. Suitable cations include sodium ion, potassium ion, magnesium ion, calcium ion, and an ammonium cation (e.g., tetramethylammonium ion). A precursor can be ester and another suitable derivative, which, during the process of preparing a polyurea or polyurethane capsule composition of this disclosure, is capable of converting to the compound, polymer, or agent and being used in preparing the polyurea or polyurethane capsule composition. A hydrate refers to the compound, polymer, or agent that contains water. A solvate refers to a complex formed between the compound, polymer, or agent and a suitable solvent. A suitable solvent can be water, ethanol, isopropanol, ethyl acetate, acetic acid, and ethanolamine.

Certain compounds, polymers, and agents have one or more stereocenters, each of which can be in the R configuration, the S configuration, or a mixture. Further, some compounds, polymers, and agents possess one or more double bonds wherein each double bond exists in the E (trans) or Z (cis) configuration, or combinations thereof. The compounds, polymers, and agents include all possible configurational stereoisomeric, regioisomeric, diastereomeric, enantiomeric, and epimeric forms as well as any mixtures thereof. As such, lysine used herein includes L-lysine, D-lysine, L-lysine monohydrochloride, D-lysine monohydrochloride, lysine carbonate, and so on. Similarly, arginine includes L-arginine, D-arginine, L-arginine monohydrochloride, D-arginine monohydrochloride, arginine carbonate, arginine monohydrate, and etc. Guanidine includes guanidine hydrochloride, guanidine carbonate, guanidine thiocyanate, and other guanidine salts including their hydrates. Ornithine include L-ornithine and its salts/hydrates (e.g., monohydrochloride) and D-ornithine and its salts/hydrates (e.g., monohydrochloride).

*Applications.* The delivery systems of the present disclosure are well-suited for use, without limitation, in the following products:
a) Household products
   i. Liquid or Powder Laundry Detergents which can use the present disclosure include those systems described in U.S. Pat. Nos. 5,929,022 and 5,916,862
   ii. Unit Dose Pouches, Tablets and Capsules such as those described in EP 1 431 382 A1, US 2013/0219996 A1, US 2013/0284637 A1, and US 6,492,315. These unit dose formulations can contain high concentrations of a functional material *(e.g.,* 5-100% fabric softening agent or detergent active), fragrance *(e.g.,* 0.5-100%, 0.5-40%, and 0.5-15%), and flavor *(e.g.,* 0.1-100%, 0.1-40%, and 1-20%). They can contain no water to limit the water content as low as less than 30% *(e.g.,* less than 20%, less than 10%, and less than 5%).
   iii. Scent Boosters such as those described in US 8,333,289 and US2014/0107010.
   iv. Fabric Care Products such as Rinse Conditioners (containing 1 to 30 weight % of a fabric conditioning active), Fabric Liquid Conditioners (containing 1 to 30 weight % of a fabric conditioning active), Tumble Drier Sheets, Fabric Refreshers, Fabric Refresher Sprays, Ironing Liquids, and Fabric Softener Systems such as those described in U.S. Pat. Nos. 6,335,315 and 5,877,145
      Liquid fabric softeners/fresheners contains at least one fabric softening agent present, preferably at a concentration of 1 to 30% (e.g., 4 to 20%, 4 to 10%, and 8 to 15%). The ratio between the active material and the fabric softening agent can be 1 : 500 to 1 : 2 (e.g., 1 : 250 to 1 : 4 and 1 : 100 to 1 :8). As an illustration, when the fabric softening agent is 5% by weight of the fabric softener, the active material is 0.01 to 2.5%, preferably 0.02 to 1.25% and more preferably 0.1 to 0.63%. As another example, when the fabric softening agent is 20% by weight of the fabric softener, the active material is 0.04 to 10%, preferably 0.08 to 5% and more preferably 0.4 to 2.5%. The active material is a fragrance, malodor counteractant or mixture thereof. The liquid fabric softener can have 0.15 to 15% of capsules (e.g., 0.5 to 10%, 0.7 to 5%, and 1 to 3%). When including capsules at these levels, the neat oil equivalent (NOE) in the softener is 0.05 to 5% (e.g., 0.15 to 3.2%, 0.25 to 2%, and 0.3 to 1%).
      Suitable fabric softening agents include cationic surfactants. Non-limiting examples are quaternary ammonium compounds such as alkylated quaternary ammonium compounds, ring or cyclic quaternary ammonium compounds, aromatic quaternary ammonium compounds, diquaternary ammonium compounds, alkoxylated quaternary ammonium compounds, amidoamine quaternary ammonium compounds, ester quaternary ammonium compounds, and mixtures thereof. Fabric softening compositions, and components thereof, are generally described in US 2004/0204337 and US 2003/0060390. Suitable softening agents include esterquats such as Rewoquat WE 18 commercially available from Evonik Industries and Stepantex SP-90 commercially available from Stepan Company.
   v. Liquid dish detergents such as those described in U.S. Pat. Nos. 6,069,122 and 5,990,065
   vi. Automatic Dish Detergents such as those described in US 6,020,294 and US6,017,871
   vii. All-purpose Cleaners including bucket dilutable cleaners and toilet cleaners
   viii. Bathroom Cleaners
   ix. Bath Tissue
   x. Rug Deodorizers
   xi. Candles
   xii. Room Deodorizers
   xiii. Floor Cleaners
   xiv. Disinfectants
   xv. Window Cleaners
   xvi. Garbage bags/trash can liners
   xvii. Air Fresheners including room deodorizer and car deodorizer, scented candles, sprays, scented oil air freshener, Automatic spray air freshener, and neutralizing gel beads
   xviii. Moisture absorber
   xix. Household Devices such as paper towels and disposable Wipes
   xx. Moth balls/traps/cakes
b) Baby Care Products
   i. Diaper Rash Cream/Balm
   ii. Baby Powder
c)Baby Care Devices
   i. Diapers
   ii. Bibs
   iii. Wipes
d) Oral Care Products. Tooth care products (as an example of preparations according to the present disclosure used for oral care) generally include an abrasive system (abrasive or polishing agent), for example silicic acids, calcium carbonates, calcium phosphates, aluminum oxides and/or hydroxylapatites, surface-active substances, for example sodium lauryl sulfate, sodium lauryl sarcosinate and/or cocamidopropylbetaine, humectants, for example glycerol and/or sorbitol, thickening agents, for example carboxymethyl cellulose, polyethylene glycols, carrageenan and/or Laponite.RTM., sweeteners, for example saccharin, taste correctors for unpleasant taste sensations, taste correctors for further, normally not unpleasant taste sensations, taste-modulating substances (for example inositol phosphate, nucleotides such as guanosine monophosphate, adenosine monophosphate or other substances such as sodium glutamate or 2-phenoxypropionic acid), cooling active ingredients, for example menthol derivatives, (for example L-menthyllactate, L-menthylalkylcarbonates, menthone ketals, menthane carboxylic acid amides), 2,2,2-trialkylacetic acid amides (for example 2,2-diisopropylpropionic acid methyl amide), icilin and icilin derivatives, stabilizers and active ingredients, for example sodium fluoride, sodium monofluorophosphate, tin difluoride, quaternary ammonium fluorides, zinc citrate, zinc sulfate, tin pyrophosphate, tin dichloride, mixtures of various pyrophosphates, triclosan, cetylpyridinium chloride, aluminum lactate, potassium citrate, potassium nitrate, potassium chloride, strontium chloride, hydrogen peroxide, flavorings and/or sodium bicarbonate or taste correctors.
   i. Tooth Paste. An exemplary formulation as follows:
      1. calcium phosphate 40-55%
      2. carboxymethyl cellulose 0.8-1.2%
      3. sodium lauryl sulfate 1.5-2.5%
      4. glycerol 20-30%
      5. saccharin 0.1-0.3%
      6. flavor oil 1-2.5%
      7. water q.s. to 100%
         A typical procedure for preparing the formulation includes the steps of (i) mixing by a blender according to the foregoing formulation to provide a toothpaste, and (ii) adding a composition of this disclosure and blending the resultant mixture till homogeneous.
   ii. Tooth Powder
   iii. Oral Rinse
   iv. Tooth Whiteners
   v. Denture Adhesive
e)Health Care Devices
   i. Dental Floss
   ii. Toothbrushes
   iii. Respirators
   iv. Scented/flavored condoms
f) Feminine Hygiene Products such as Tampons, Feminine Napkins and Wipes, and Pantiliners
g) Personal Care Products: Cosmetic or pharmaceutical preparations, e.g., a "water-in-oil" (W/O) type emulsion, an "oil-in-water" (O/W) type emulsion or as multiple emulsions, for example of the water-in-oil-in-water (W/O/W) type, as a PIT emulsion, a Pickering emulsion, a micro-emulsion or nano-emulsion; and emulsions which are particularly preferred are of the "oil-in-water" (O/W) type or water-in-oil-in-water (W/O/W) type. More specifically,
   i. Personal Cleansers (bar soaps, body washes, and shower gels)
   ii. In-shower conditioner
   iii. Sunscreen ant tattoo color protection (sprays, lotions, and sticks)
   iv. Insect repellants
   v. Hand Sanitizer
   vi. Antiinflammatory balms, ointments, and sprays
   vii. Antibacterial ointments and creams
   viii. Sensates
   ix. Deodorants and Antiperspirants including aerosol and pump spray antiperspirant, stick antiperspirant, roll-on antiperspirant, emulsion spray antiperspirant, clear emulsion stick antiperspirant, soft solid antiperspirant, emulsion roll-on antiperspirant, clear emulsion stick antiperspirant, opaque emulsion stick antiperspirant, clear gel antiperspirant, clear stick deodorant, gel deodorant, spray deodorant, roll-on, and cream deordorant.
   x. Wax-based Deodorant. An exemplary formulation as follows:
      1.Parafin Wax 10-20%
      2.Hydrocarbon Wax 5-10%
      3.White Petrolatum 10-15%
      4. Acetylated Lanolin Alcohol 2-4%
      5.Diisopropyl Adipate 4-8%
      6. Mineral Oil 40-60%
      7. Preservative (as needed)
         The formulation is prepared by (i) mixing the above ingredients, (ii) heating the resultant composition to 75 °C until melted, (iii) with stirring, adding 4% cryogenically ground polymer containing a fragrance while maintaining the temperature 75 °C, and (iv) stirring the resulting mixture in order to ensure a uniform suspension while a composition of this disclosure is added to the formulation.
   xi. Glycol/Soap Type Deodorant. An exemplary formulation as follows:
      1.Propylene Glycol 60-70%
      2. Sodium Stearate 5-10%
      3. Distilled Water 20-30%
      4.2,4,4-Trichloro-2'- Hydroxy Diphenyl Ether, manufactured by the Ciba-Geigy Chemical Company and a Trademark of the Ciba-Geigy Chemical Company) 0.01-0.5%
         The ingredients are combined and heated to 75 °C with stirring until the sodium stearate has dissolved. The resulting mixture is cooled to 40 °C followed by addition of a composition of this disclosure.
   xii. Lotion including body lotion, facial lotion, and hand lotion
   xiii. Body powder and foot powder
   xiv. Toiletries
   xv. Body Spray
   xvi. Shave cream and male grooming products
   xvii. Bath Soak
   xviii. Exfoliating Scrub
h) Personal Care Devices
   i. Facial Tissues
   ii. Cleansing wipes
i) Hair Care Products
   i. Shampoos (liquid and dry powder)
   ii. Hair Conditioners (Rinse-out conditioners, leave-in conditioners, and cleansing conditioners)
   iii. Hair Rinses
   iv. Hair Refreshers
   v. Hair perfumes
   vi. Hair straightening products
   vii. Hair styling products, Hair Fixative and styling aids
   viii. Hair combing creams
   ix. Hair wax
   x. Hair foam, hair gel, nonaerosol pump spray
   xi. Hair Bleaches, Dyes and Colorants
   xii. Perming agents
   xiii. Hair wipes
j) Beauty Care
   i. Fine Fragrance - Alcoholic. Compositions and methods for incorporating fragrance capsules into alcoholic fine fragrances are described in US 4,428,869. Alcoholic fine fragrances may contain the following:
      1. Ethanol (1-99%)
      2. Water (0-99%)
      3.A suspending aide including but not limited to: hydroxypropyl cellulose, ethyl cellulose, silica, microcrystalline cellulose, carrageenan, propylene glycol alginate, methyl cellulose, sodium carboxymethyl cellulose or xanthan gum (0.-1-%)
      4. Optionally an emulsifier or an emollient may be included including but not limited to those listed above
   ii. Solid Perfume
   iii. Lipstick/lip balm
   iv. Make-up cleanser
   v. Skin care cosmetic such as foundation, pack, sunscreen, skin lotion, milky lotion, skin cream, emollients, skin whitening
   vi. Make-up cosmetic including manicure, mascara, eyeliner, eye shadow, liquid foundation, powder foundation, lipstick and cheek rouge
k) Consumer goods packaging such as fragranced cartons, fragranced plastic bottles/boxes
l) Pet care products
   i. Cat litter
   ii. Flea and tick treatment products
   iii. Pet grooming products
   iv. Pet shampoos
   v. Pet toys, treats, and chewables
   vi. Pet training pads
   vii. Pet carriers and crates
m) Confectionaries confectionery, preferably selected from the group consisting of chocolate, chocolate bar products, other products in bar form, fruit gums, hard and soft caramels and chewing gum
   i. Gum
      1.Gum base (natural latex chicle gum, most current chewing gum bases also presently include elastomers, such as polyvinylacetate (PVA), polyethylene, (low or medium molecular weight) polyisobutene (PIB), polybutadiene, isobutene-isoprene copolymers (butyl rubber), polyvinylethylether (PVE), polyvinylbutyether, copolymers of vinyl esters and vinyl ethers, styrene-butadiene copolymers (styrene-butadiene rubber, SBR), or vinyl elastomers, for example based on vinylacetate/vinyllaurate, vinylacetate/vinylstearate or ethylene/vinylacetate, as well as mixtures of the mentioned elastomers, as described for example in EP 0 242 325, U.S. Pat. No. 4,518,615, U.S. Pat. No. 5,093,136, U.S. Pat. No. 5,266,336, U.S. Pat. No. 5,601,858 or U.S. Pat. No. 6,986,709.) 20-25%
      2. Powdered sugar 45-50%
      3.glucose 15-17%
      4. starch syrup 10-13%
      5.plasticizer 0.1%
      6.flavor 0.8-1.2%
         The components described above were kneaded by a kneader according to the foregoing formulation to provide a chewing gum. Encapsulated Flavor or sensate is then added and blended till homogeneous.
   ii. Breath Fresheners
   iii. Orally Dissolvable Strips
   iv. Chewable Candy
   v. Hard Candy
n) Baked products, preferably selected from the group consisting of bread, dry biscuits, cakes and other cookies;
o) snack foods, preferably selected from the group consisting of baked or fried potato chips or potato dough products, bread dough products and corn or peanut-based extrudates;
   i. Potato, tortilla, vegetable or multigrain chips
   ii. Popcorn
   iii. Pretzels
   iv. Extruded stacks
p) Cereal Products preferably selected from the group consisting of breakfast cereals, muesli bars and precooked finished rice products
q) Alcoholic and non-alcoholic beverages, preferably selected from the group consisting of coffee, tea, wine, beverages containing wine, beer, beverages containing beer, liqueurs, schnapps, brandies, sodas containing fruit, isotonic beverages, soft drinks, nectars, fruit and vegetable juices and fruit or vegetable preparations; instant beverages, preferably selected from the group consisting of instant cocoa beverages, instant tea beverages and instant coffee beverages
   i. Ready to drink liquid drinks
   ii. Liquid Drink Concentrates
   iii. Powder Drinks
   iv. Coffee: Instant Cappucino
      1. Sugar 30-40%
      2.Milk Powder 24-35%
      3. Soluble Coffee 20-25%
      4.Lactose 1-15%
      5.Food Grade Emulsifier 1-3%
      6.Encapsulated Volatile Flavor 0.01-0.5%
   v. Tea
   vi. Alcoholic
r) Spice blends and consumer prepared foods
   i. Powder gravy, sauce mixes
   ii. Condiments
   iii. Fermented Products
s) Ready to heat foods: ready meals and soups, preferably selected from the group consisting of powdered soups, instant soups, precooked soups
   i. Soups
   ii. Sauces
   iii. Stews
   iv. Frozen entrees
t) Dairy Products milk products, preferably selected from the group consisting of milk beverages, ice milk, yogurt, kefir, cream cheese, soft cheese, hard cheese, powdered milk, whey, butter, buttermilk and partially or fully hydrolyzed milk protein-containing products Flavored milk beverages
   i. Yoghurt
   ii. Ice cream
   iii. Bean Curd
   iv. Cheese
u) Soya protein or other soybean fractions, preferably selected from the group consisting of soya milk and products produced therefrom, soya lecithin-containing preparations, fermented products such as tofu or tempeh or products produced therefrom and soy sauces;
v) Meat products, preferably selected from the group consisting of ham, fresh or raw sausage preparations, and seasoned or marinated fresh or salt meat products
w) Eggs or egg products, preferably selected from the group consisting of dried egg, egg white and egg yolk
x) Oil-based products or emulsions thereof, preferably selected from the group consisting of mayonnaise, remoulade, dressings and seasoning preparations
y) fruit preparations, preferably selected from the group consisting of jams, sorbets, fruit sauces and fruit fillings; vegetable preparations, preferably selected from the group consisting of ketchup, sauces, dried vegetables, deep-frozen vegetables, precooked vegetables, vegetables in vinegar and preserved vegetables
z)Flavored pet foods.

The above-listed applications are all well known in the art. For example, fabric softener systems are described in US Patent Nos. 6,335,315 and 5,674,832. Liquid laundry detergents include those systems described in U.S. Patent Nos. 5,929,022 and 5,916,862. Liquid dish detergents are described in U.S. Patent Nos. 6,069,122 and 5,990,065. Shampoo and conditioners that can employ the present disclosure include those described in US Patent Nos. 6,162,423 and 5,968,286. Automatic Dish Detergents are described in U.S. Pat. Nos. 6,020,294 and 6,017,871.

The invention is described in greater detail by the following non-limiting examples. Without further elaboration, it is believed that one skilled in the art can, based on the description herein, utilize the present invention to its fullest extent.

### EXAMPLE 1

A BPEI microcapsule composition, Composition 1, was prepared by crosslinking BPEI and glutaraldehyde, a dialdehyde having the formula CH₂(CH₂CHO)₂.

First, an oil phase was prepared by mixing 96 grams of fragrance oil and 24 grams of Neobee oil. An aqueous phase was prepared in a separate container by mixing 15 grams of polyvinyl pyrrolidone and 60 grams of Luviquat PQ 11 (polyquaternium-11) in 41.4 grams of water. The water phase was then added to the oil phase. The resultant mixture was homogenized at 12500 RPM for 2 min and continuously mixed with an overhead stirrer for 2 minutes to obtain an oil-in-water emulsion, to which 25.2 grams of 3% BPEI solution in water was slowly added, followed by the addition of 38.4 grams of 2.5% glutaraldehyde. Subsequently, the mixture was stirred for 30 minutes to allow the formation of a microcapsule precursor. Curing at 55 °C for 4 to 5 hours gave Microcapsule 1.

Microcapsule 1 was observed by optical microscopy. The particle sizes of the microcapsules were measured and found to be in the range of 0.5 to 80 µm.

### REFERENCE EXAMPLE 2

A second microcapsule composition, *i.e.,* Composition 2, was prepared by coacervate BPEI into an aggregate to encapsulate a fragrance oil.

Thirty three grams of a fragrance, *i.e.,* Eden AI (commercially available from International Flavors and Fragrance, Union Beach, NJ) was weighed out as an oil phase. In a separate beaker, an aqueous solution (57 grams) containing 1.8% of FLEXAN II (Akzo Nobel, Bridgewater, NJ) was mixed with a solution (10 grams) of 1% CMC in water to obtain an aqueous phase. The oil phase was then emulsified into the aqueous phase to form an oil-in-water emulsion under shearing (ULTRA TURRAX, T25 Basic, IKA WERKE) at 6500 rpm for two minutes.

Subsequently, a 5.6 mL aqueous solution with 18 wt% poly(Poly(4-styrenesulfonic acid) (PSS, molecular weight of 75,000 g/mol) was slowly added into the emulsion under agitation. After 30 minutes, 3.3 mL of 30% branched polyethyleneimine (BPEI, Sigma-Aldrich, St. Louis, MO) was added and stirred for 30 minutes. Then, 5 mL of 20% Gelatin (Sigma-Aldrich, St. Louis, MO) was added under constant mixing with an overhead mixer. This cycle of adding the PSS solution, the BPEI solution, and then gelatin solution was repeated five times to obtain a BPEI microcapsule having 5 layers of PSS-BPEI-gelatin aggregate that encapsulating a fragrance oil core.

## Claims

1. A microcapsule comprising an oil core and a microcapsule wall encapsulating the oil core, wherein
the oil core contains an active material,
the microcapsule wall is formed of an encapsulating polymer that is a reaction product of (i) a branched polyethyleneimine, a mixture of the branched polyethyleneimine and a polyfunctional amine, or a mixture of the branched polyethyleneimine and a polyfunctional alcohol, and (ii) a carbonyl crosslinker, or a mixture of a carbonyl crosslinker and a polyisocyanate,
the carbonyl crosslinker has a first functional group and a second functional group,
the first functional group is a carbonyl electrophilic group reactive towards the branched polyethyleneimine, the polyfunctional amine, or the polyfunctional alcohol,
the second functional group is an electrophilic group reactive towards the branched polyethyleneimine, the polyfunctional amine, or the polyfunctional alcohol,
the branched polyethyleneimine has a molecular weight of 750 to 50,000 Daltons,
the carbonyl crosslinker has a molecular weight of 50 to 2,500 Daltons,
the molar ratio between the branched polyethyleneimine and the carbonyl crosslinker is 1 : 20 to 20 : 1,
the first functional group is formyl, an acyl halide group, a carboxylic anhydride group, and
the second functional group is formyl, keto, carboxyl, a carboxylate ester group, an acyl halide group, an amide group, a carboxylic anhydride group, an alkyl halide group, an epoxide group, an aziridine group, an oxetane group, an azetidine group, a sulfonyl halide group, a chlorophosphate group, an isocyanate group, an α,β-unsaturated carbonyl group, an α,β-unsaturated nitrile group, or an α,β-unsaturated methanesulfonyl group.

2. The microcapsule of claim 1, wherein the carbonyl crosslinker is glyoxal, 1,3-propane dialdehyde, 1,4-butane dialdehyde, 1,5-pentane dialdehyde, 1,6-hexane dialdehyde, starch aldehyde, or a combination thereof.

3. The microcapsule of any preceding claim, further comprising a deposition aid selected from the group consisting of polyquaternium-4, polyquaternium-5, polyquaternium-6, polyquaternium-7, polyquaternium-10, polyquaternium-16, polyquaternium-22, polyquaternium-24, polyquaternium-28, polyquaternium-39, polyquaternium-44, polyquaternium-46, polyquaternium-47, polyquaternium-53, polyquaternium-55, polyquaternium-67, polyquaternium-68, polyquaternium-69, polyquaternium-73, polyquaternium-74, polyquaternium-77, polyquaternium-78, polyquaternium-79, polyquaternium-80, polyquaternium-81, polyquaternium-82, polyquaternium-86, polyquaternium-88, polyquaternium-101, polyvinylamine, polyethyleneimine, polyvinylamine and vinylformamide copolymer, and combinations thereof; wherein the active material is a fragrance, pro-fragrance, flavor, vitamin or derivative thereof, malodor counteractive agent, anti-inflammatory agent, fungicide, anesthetic, analgesic, antimicrobial active, anti-viral agent, anti-infectious agent, anti-acne agent, skin lightening agent, insect repellant, emollient, skin moisturizing agent, wrinkle control agent, UV protection agent, fabric softener active, hard surface cleaning active, skin or hair conditioning agent, insect repellant, animal repellent, vermin repellent, flame retardant, antistatic agent, nanometer to micron size inorganic solid, polymeric or elastomeric particle, or combination thereof.

4. A method of preparing a microcapsule as defined in claim 1, the method comprising:
(a) providing an oil-in-water emulsion containing (i) a branched polyethyleneimine, a mixture of the branched polyethyleneimine and a polyfunctional amine, or a mixture of the branched polyethyleneimine and a polyfunctional alcohol, (ii) a carbonyl crosslinker, or a mixture of the carbonyl crosslinker and a polyisocyanate, (iii) an oil phase having an active material, and (iv) an aqueous phase having a microcapsule formation aid and water,
(b) causing the formation of a microcapsule precursor having an oil core that contains the active material and a microcapsule wall that is formed of an encapsulating polymer, and
(c) curing the microcapsule precursor to obtain a microcapsule slurry containing the microcapsule,
wherein
the carbonyl crosslinker contains a first functional group and a second functional group,
the first functional group is a carbonyl electrophilic group,
the second functional group is an electrophilic group,
the branched polyethyleneimine having a molecular weight of 750 to 25,000,
the carbonyl crosslinker has a molecular weight of 50 to 2,500,
the molar ratio between the polyfunctional amine and the carbonyl crosslinker is 1 : 20 to 20 : 1,
the first functional group is formyl, an acyl halide group, a carboxylic anhydride group,
the second functional group is formyl, keto, carboxyl, a carboxylate ester group, an acyl halide group, an amide group, a carboxylic anhydride group, an alkyl halide group, an epoxide group, an aziridine group, an oxetane group, an azetidine group, a sulfonyl halide group, a chlorophosphate group, an isocyanate group, an α,β-unsaturated carbonyl group, an α,β-unsaturated nitrile group, or an α,β-unsaturated methanesulfonyl group,
the microcapsule formation aid is polyvinyl pyrrolidone, polyvinyl alcohol, poly(styrene sulfonate), carboxymethyl cellulose, sodium salt of naphthalene sulfonate condensate, co-polymer of ethylene and maleic anhydride, an alginate, hyaluronic acid, poly(acrylic acid), carboxymethylecellulose, copolymers of acrylic acid and acrylamide, copolymer of acrylamide and acrylamidopropyltrimonium chloride, terpolymers of (acrylic acid, acrylamide, and acrylamidopropyltrimonium chloride), partially or completely hydrolyzed polyvinyl acetate polymers, or a combination thereof, and
the microcapsule precursor is cured at a temperature of 20 to 250 °C.

5. The method of claim 4, further comprising the step of spray drying the microcapsule slurry to obtain the microcapsule in a powder form.

6. The microcapsule of any one of claims 1 to 3, or the method of any one of claims 4 to 5, wherein the active material is a fragrance or a flavor.

7. A consumer product comprising a microcapsule as defined in any one of claims 1 to 3 and 6.

8. The consumer product of claim 7, wherein the consumer product is a hair care product, a personal care product, a fabric care product, or a home care product.

## Patentansprüche

1. Mikrokapsel, umfassend einen Ölkern und eine Mikrokapselwand, die den Ölkern einkapselt, wobei
der Ölkern ein aktives Material enthält,
die Mikrokapselwand aus einem einkapselnden Polymer gebildet ist, das ein Reaktionsprodukt ist aus (i) einem verzweigtem Polyethylenimin, einem Gemisch aus dem verzweigten Polyethylenimin und einem polyfunktionellen Amin oder einem Gemisch aus dem verzweigten Polyethylenimin und einem polyfunktionellen Alkohol, und (ii) einem Carbonylvernetzer oder einem Gemisch aus einem Carbonylvernetzer und einem Polyisocyanat,
der Carbonylvernetzer eine erste Funktionsgruppe und eine zweite Funktionsgruppe aufweist,
die erste Funktionsgruppe eine elektrophile Carbonylgruppe ist, die gegenüber dem verzweigten Polyethylenimin, dem polyfunktionellen Amin oder dem polyfunktionellen Alkohol reaktiv ist,
die zweite Funktionsgruppe eine elektrophile Gruppe ist, die gegenüber dem verzweigten Polyethylenimin, dem polyfunktionellen Amin oder dem polyfunktionellen Alkohol reaktiv ist,
das verzweigte Polyethylenimin ein Molekulargewicht von 750 bis 50.000 Dalton hat,
der Carbonylvernetzer ein Molekulargewicht von 50 bis 2.500 Dalton hat,
das molare Verhältnis zwischen dem verzweigten Polyethylenimin und dem Carbonylvernetzer 1 : 20 bis 20 : 1 beträgt,
die erste Funktionsgruppe Formyl, eine Acylhalogenidgruppe, eine Carbonsäureanhydridgruppe ist und
die zweite Funktionsgruppe Formyl, Keto, Carboxyl, eine Carboxylatestergruppe, eine Acylhalogenidgruppe, eine Amidgruppe, eine Carbonsäureanhydridgruppe, eine Alkylhalogenidgruppe, eine Epoxidgruppe, eine Aziridingruppe, eine Oxetangruppe, eine Azetidingruppe, eine Sulfonylhalogenidgruppe, eine Chlorphosphatgruppe, eine Isocyanatgruppe, eine α,β-ungesättigte Carbonylgruppe, eine α,β-ungesättigte Nitrilgruppe oder eine α,β-ungesättigte Methansulfonylgruppe ist.

2. Mikrokapsel nach Anspruch 1, wobei der Carbonylvernetzer Glyoxal, 1,3-Propandialdehyd, 1,4-Butandialdehyd, 1,5-Pentandialdehyd, 1,6-Hexandialdehyd, Stärkealdehyd oder eine Kombination davon ist.

3. Mikrokapsel nach einem der vorhergehenden Ansprüche, ferner umfassend ein Abscheidungshilfsmittel, das aus der Gruppe bestehend aus Polyquatemium-4, Polyquatemium-5, Polyquatemium-6, Polyquatemium-7, Polyquatemium-10, Polyquatemium-16, Polyquatemium-22, Polyquatemium-24, Polyquatemium-28, Polyquatemium-39, Polyquatemium-44, Polyquatemium-46, Polyquatemium-47, Polyquatemium-53, Polyquatemium-55, Polyquatemium-67, Polyquatemium-68, Polyquatemium-69, Polyquatemium-73, Polyquatemium-74, Polyquatemium-77, Polyquatemium-78, Polyquatemium-79, Polyquatemium-80, Polyquatemium-81, Polyquatemium-82, Polyquatemium-86, Polyquatemium-88, Polyquatemium-101, Polyvinylamin, Polyethylenimin, Polyvinylamin und Vinylformamid-Copolymer und Kombinationen davon ausgewählt ist; wobei das aktive Material ein Duftstoff, Pro-Duftstoff, ein Geschmacksstoff, ein Vitamin oder ein Derivat davon, ein geruchshemmendes Mittel, ein entzündungshemmendes Mittel, ein Fungizid, ein Anästhetikum, ein Analgetikum, ein antimikrobieller Wirkstoff, ein antivirales Mittel, ein Mittel gegen Infektionen, ein Mittel gegen Akne, ein hautaufhellendes Mittel, ein Insektenabwehrmittel, ein Weichmacher, ein hautbefeuchtendes Mittel, ein Mittel gegen Falten, ein UV-Schutzmittel, ein Weichspülmittel, ein Reinigungsmittel für harte Oberflächen, ein Haut- oder Haarkonditionierungsmittel, ein Insektenabwehrmittel, ein Tierabwehrmittel, ein Ungezieferabwehrmittel, ein Flammschutzmittel, ein antistatisches Mittel, anorganische Feststoff-, Polymer- oder Elastomerteilchen in Nanometer- bis Mikrometergröße oder eine Kombination davon ist.

4. Verfahren zur Herstellung einer Mikrokapsel nach Anspruch 1, wobei das Verfahren umfasst:
(a) Bereitstellen einer Öl-in-Wasser-Emulsion, die (i) ein verzweigtes Polyethylenimin, ein Gemisch aus dem verzweigten Polyethylenimin und einem polyfunktionellen Amin oder ein Gemisch aus dem verzweigten Polyethylenimin und einem polyfunktionellen Alkohol, (ii) einen Carbonylvernetzer oder ein Gemisch aus dem Carbonylvernetzer und einem Polyisocyanat, (iii) eine Ölphase mit einem aktiven Material und (iv) eine wässrige Phase mit einem Mikrokapselbildungshilfsmittel und Wasser enthält,
(b) Bewirken der Bildung eines Mikrokapselvorläufers mit einem Ölkern, der das aktive Material enthält, und einer aus einem einkapselnden Polymer gebildeten Mikrokapselwand, und
(c) Härten des Mikrokapselvorläufers, um eine Mikrokapselaufschlämmung zu erhalten, die die Mikrokapsel enthält,
wobei
der Carbonylvernetzer eine erste Funktionsgruppe und eine zweite Funktionsgruppe enthält,
die erste Funktionsgruppe eine elektrophile Carbonylgruppe ist,
die zweite Funktionsgruppe eine elektrophile Gruppe ist,
das verzweigte Polyethylenimin ein Molekulargewicht von 750 bis 25.000 hat,
der Carbonylvernetzer ein Molekulargewicht von 50 bis 2.500 hat,
das molare Verhältnis zwischen dem polyfunktionellen Amin und dem Carbonylvernetzer 1 : 20 bis 20 : 1 beträgt,
die erste Funktionsgruppe Formyl, eine Acylhalogenidgruppe, eine Carbonsäureanhydridgruppe ist,
die zweite Funktionsgruppe Formyl, Keto, Carboxyl, eine Carboxylatestergruppe, eine Acylhalogenidgruppe, eine Amidgruppe, eine Carbonsäureanhydridgruppe, eine Alkylhalogenidgruppe, eine Epoxidgruppe, eine Aziridingruppe, eine Oxetangruppe, eine Azetidingruppe, eine Sulfonylhalogenidgruppe, eine Chlorphosphatgruppe, eine Isocyanatgruppe, eine α,β-ungesättigte Carbonylgruppe, eine α,β-ungesättigte Nitrilgruppe oder eine α,β-ungesättigte Methansulfonylgruppe ist,
das Mikrokapselbildungshilfsmittel Polyvinylpyrrolidon, Polyvinylalkohol, Poly(styrolsulfonat), Carboxymethylcellulose, Natriumsalz von Naphthalinsulfonatkondensat, ein Copolymer aus Ethylen und Maleinsäureanhydrid, ein Alginat, Hyaluronsäure, Poly(acrylsäure), Carboxymethylecellulose, Copolymere aus Acrylsäure und Acrylamid, ein Copolymer aus Acrylamid und Acrylamidopropyltrimoniumchlorid, Terpolymere aus (Acrylsäure, Acrylamid und Acrylamidopropyltrimoniumchlorid), teilweise oder vollständig hydrolysierte Polyvinylacetatpolymere oder eine Kombination davon ist, und
der Mikrokapselvorläufer bei einer Temperatur von 20 bis 250 °C gehärtet wird.

5. Verfahren nach Anspruch 4, ferner umfassend den Schritt der Sprühtrocknung der Mikrokapselaufschlämmung, um die Mikrokapsel in Pulverform zu erhalten.

6. Mikrokapsel nach einem der Ansprüche 1 bis 3 oder das Verfahren nach einem der Ansprüche 4 bis 5, wobei das aktive Material ein Duftstoff oder Geschmacksstoff ist.

7. Verbraucherprodukt, umfassend eine Mikrokapsel nach einem der Ansprüche 1 bis 3 und 6.

8. Verbraucherprodukt nach Anspruch 7, wobei das Verbraucherprodukt ein Haarpflegeprodukt, ein Körperpflegeprodukt, ein Textilpflegeprodukt oder ein Haushaltspflegeprodukt ist.

## Revendications

1. Microcapsule comprenant un cœur huileux et une paroi de microcapsule encapsulant le cœur huileux, dans laquelle
le cœur huileux contient une matière active,
la paroi de microcapsule est formée d'un polymère d'encapsulation qui est un produit de réaction de (i) une polyéthylèneimine ramifiée, un mélange de la polyéthylèneimine ramifiée et d'une amine polyfonctionnelle, ou un mélange de la polyéthylèneimine ramifiée et d'un alcool polyfonctionnel, et (ii) un agent de réticulation carbonyle, ou un mélange d'un agent de réticulation carbonyle et d'un polyisocyanate,
l'agent de réticulation carbonyle possède un premier groupe fonctionnel et un second groupe fonctionnel,
le premier groupe fonctionnel est un groupe électrophile carbonyle capable de réagir envers la polyéthylèneimine ramifiée, l'amine polyfonctionnelle ou l'alcool polyfonctionnel,
le second groupe fonctionnel est un groupe électrophile capable de réagir envers la polyéthylèneimine ramifiée, l'amine polyfonctionnelle ou l'alcool polyfonctionnel,
la polyéthylèneimine ramifiée a une masse moléculaire de 750 à 50 000 Daltons,
l'agent de réticulation carbonyle a une masse moléculaire de 50 à 2 500 Daltons,
le rapport molaire entre la polyéthylèneimine ramifiée et l'agent de réticulation carbonyle est de 1:20 à 20:1,
le premier groupe fonctionnel est formyle, un groupe halogénure d'acyle, un groupe anhydride carboxylique, et
le second groupe fonctionnel est formyle, céto, carboxyle, un groupe ester carboxylate, un groupe halogénure d'acyle, un groupe amide, un groupe anhydride carboxylique, un groupe halogénure d'alkyle, un groupe époxyde, un groupe aziridine, un groupe oxétane, un groupe azétidine, un groupe halogénure de sulfonyle, un groupe chlorophosphate, un groupe isocyanate, un groupe carbonyle α,β-insaturé, un groupe nitrile α,β-insaturé ou un groupe méthanesulfonyle α,β-insaturé.

2. Microcapsule selon la revendication 1, dans laquelle l'agent de réticulation carbonyle est le glyoxal, le 1,3-propane dialdéhyde, le 1,4-butane dialdéhyde, le 1,5-pentane dialdéhyde, le 1,6-hexane dialdéhyde, l'amidon aldéhyde, ou une combinaison de ceux-ci.

3. Microcapsule selon l'une quelconque des revendications précédentes, comprenant en outre un auxiliaire de dépôt choisi dans le groupe constitué par le polyquaternium-4, le polyquaternium-5, le polyquaternium-6, le polyquaternium-7, le polyquaternium-10, le polyquaternium-16, le polyquaternium-22, le polyquaternium-24, le polyquaternium-28, le polyquaternium-39, le polyquaternium-44, le polyquaternium-46, le polyquaternium-47, le polyquaternium-53, le polyquaternium-55, le polyquaternium-67, le polyquaternium-68, le polyquaternium-69, le polyquaternium-73, le polyquaternium-74, le polyquaternium-77, le polyquaternium-78, le polyquaternium-79, le polyquaternium-80, le polyquaternium-81, le polyquaternium-82, le polyquaternium-86, le polyquaternium-88, le polyquaternium-101, une polyvinylamine, une polyéthylèneimine, une polyvinylamine et un copolymère de vinylformamide, et leurs combinaisons ; dans laquelle la matière active est un parfum, un précurseur de parfum, un arôme, une vitamine ou un dérivé de ceux-ci, un agent aigssant contre les mauvaises odeurs, un agent antiinflammatoire, un fongicide, un anesthésique, un analgésique, un actif antimicrobien, un agent antiviral, un agent anti-infectieux, un agent anti-acné, un agent d'éclaircissement de la peau, un produit anti-insectes, un émollient, un agent d'hydratation de la peau, un agent de lutte contre les rides, un agent de protection contre les UV, un agent assouplissant pour tissus, un agent de nettoyage des surfaces dures, un agent de conditionnement de la peau ou des cheveux, un répulsif pour insectes, un répulsif pour animaux, un répulsif pour vermine, un retardateur de flamme, un agent antistatique, un solide inorganique de dimension manométrique à micrométrique, une particule polymère ou élastomère, ou une combinaison de ceux-ci.

4. Procédé de préparation d'une microcapsule telle que définie dans la revendication 1, le procédé comprenant :
a) fournir une émulsion huile-dans-eau contenant (i) une polyéthylèneimine ramifiée, un mélange de la polyéthylèneimine ramifiée et d'une amine polyfonctionnelle, ou un mélange de la polyéthylèneimine ramifiée et d'un alcool polyfonctionnel, (ii) un agent de réticulation carbonyle, ou un mélange de l'agent de réticulation carbonyle et d'un polyisocyanate, (iii) une phase huileuse ayant une matière active et (iv) une phase aqueuse ayant un auxiliaire de formation de microcapsule et de l'eau ;
b) provoquer la formation d'un précurseur de microcapsule ayant un coeur huileux qui contient la matière active et une paroi de microcapsule qui est formée d'un polymère d'encapsulation ; et
c) faire durcir le précurseur de microcapsule pour obtenir une bouillie de microcapsules contenant la microcapsule, où
l'agent de réticulation carbonyle contient un premier groupe fonctionnel et un second groupe fonctionnel,
le premier groupe fonctionnel est un groupe électrophile carbonyle,
le second groupe fonctionnel est un groupe électrophile,
la polyéthylèneimine ramifiée a une masse moléculaire de 750 à 25 000,
l'agent de réticulation carbonyle a une masse moléculaire de 50 à 2 500,
le rapport molaire entre l'amine polyfonctionnelle et l'agent de réticulation carbonyle est de 1:20 à 20:1,
le premier groupe fonctionnel est formyle, un groupe halogénure d'acyle, un groupe anhydride carboxylique,
le second groupe fonctionnel est formyle, céto, carboxyle, un groupe ester carboxylate, un groupe halogénure d'acyle, un groupe amide, un groupe anhydride carboxylique, un groupe halogénure d'alkyle, un groupe époxyde, un groupe aziridine, un groupe oxétane, un groupe azétidine, un groupe halogénure de sulfonyle, un groupe chlorophosphate, un groupe isocyanate, un groupe carbonyle α,β-insaturé, un groupe nitrile α,β-insaturé ou un groupe méthanesulfonyle α,β-insaturé,
l'auxiliaire de formation de microcapsule est la polyvinyl pyrrolidone, l'alcool polyvinylique, le poly(styrène sulfonate), la carboxyméthyl cellulose, le sel de sodium d'un condensat de naphtalène sulfonate, un copolymère d'éthylène et d'anhydride maléique, un alginate, l'acide hyaluronique, l'acide polyacrylique, la carboxyméthylcellulose, les copolymères d'acide acrylique et d'acrylamide, un copolymère d'acrylamide et de chlorure d'acrylamidopropyltrimonium, les terpolymères d'(acide acrylique, acrylamide et chlorure d'acrylamidopropyltrimonium), les polymères d'acétate de polyvinyle partiellement ou complètement hydrolysés, ou une combinaison de ceux-ci, et
le précurseur de microcapsule est durci à une température de 20 à 250°C.

5. Procédé selon la revendication 4, comprenant en outre l'étape de séchage par pulvérisation de la bouillie de microcapsules pour obtenir la microcapsule sous une forme de poudre.

6. Microcapsule selon l'une quelconque des revendications 1 à 3, ou procédé selon l'une quelconque des revendications 4 à 5, dans laquelle / dans lequel la matière active est un parfum ou un arôme.

7. Produit de consommation comprenant une microcapsule telle que définie dans l'une quelconque des revendications 1 à 3 et 6.

8. Produit de consommation selon la revendication 7, dans lequel le produit de consommation est un produit de soins capillaires, un produit de soins personnels, un produit d'entretien des tissus, ou un produit d'entretien ménager.
